(19) Europäisches Patentamt
European Patent Office
Office européen des brevets

(11) **EP 2 788 011 B1**

(12) **EUROPEAN PATENT SPECIFICATION**

(45) Date of publication and mention of the grant of the patent:
**16.05.2018 Bulletin 2018/20**

(21) Application number: **12815804.5**

(22) Date of filing: **05.12.2012**

(51) Int Cl.:
*A61K 36/738* *(2006.01)*   *A61K 36/48* *(2006.01)*
*A61K 36/66* *(2006.01)*   *A61P 17/00* *(2006.01)*
*A61P 17/02* *(2006.01)*   *A61P 17/06* *(2006.01)*
*A61P 17/08* *(2006.01)*   *A61P 17/10* *(2006.01)*
*A61P 17/12* *(2006.01)*

(86) International application number:
**PCT/IB2012/056988**

(87) International publication number:
**WO 2013/084163 (13.06.2013 Gazette 2013/24)**

(54) **COMPOSITION COMPRISING A CHELIDONIUM MAJUS EXTRACT AND COPAIBA, AND THE USE THEREOF FOR THE TREATMENT OF CUTANEOUS DYSFUNCTIONS**

ZUSAMMENSETZUNG MIT EINEM CHELIDONIUM-MAJUS-EXTRAKT UND COPAIBA SOWIE VERWENDUNG DAVON ZUR BEHANDLUNG KUTANER DYSFUNKTIONEN

COMPOSITION COMPRENANT UN EXTRAIT DE CHELIDONIUM MAJUS ET DU COPAHU, ET UTILISATION DE CELLE-CI POUR TRAITER DES DYSFONCTIONNEMENTS CUTANÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2011 IT MI20112216**

(43) Date of publication of application:
**15.10.2014 Bulletin 2014/42**

(73) Proprietors:
• **Fondazione Salvatore Maugeri Clinica del Lavoro e della Riabilitazione**
  **27100 Pavia (IT)**
• **PURYTRA FARMACEUTICI S.P.A.**
  **20135 Milano (IT)**

(72) Inventors:
• **PIETRA, Daniele**
  **I-56017 San Giuliano Terme (IT)**
• **BORGHINI, Alice**
  **I-56017 San Giuliano Terme (IT)**
• **DEL CORSO, Simone**
  **I-56122 Pisa (IT)**
• **IMBRIANI, Marcello**
  **I-27100 Pavia (IT)**
• **BIANUCCI, Anna**
  **I-56122 Pisa (IT)**

(74) Representative: **Zaccaro, Elisabetta et al Notarbartolo & Gervasi S.p.A. Corso di Porta Vittoria, 9 20122 Milano (IT)**

(56) References cited:
**WO-A1-2009/112226      DE-A1- 4 031 960**
**DE-U1- 20 314 864       FR-A1- 2 661 330**
**JP-A- 8 012 560**

• **PAIVA L A F ET AL: "Investigation on the wound healing activity of oleo-resin from Copaifera langsdorffi in rats", MEDICINAL & AROMATIC PLANTS ABSTRACTS, SCIENTIFIC PUBLISHERS, SCIENTIFIC PUBLISHERS, NEW DELHI - INDIA, vol. 25, no. 5, 1 October 2003 (2003-10-01), XP018009972, ISSN: 0250-4367**
• **VEIGA ET AL: "Chemical composition and anti-inflammatory activity of copaiba oils from Copaifera cearensis Huber ex Ducke, Copaifera reticulata Ducke and Copaifera multijuga Hayne-A comparative study", JOURNAL OF ETHNOPHARMACOLOGY, ELSEVIER SCIENTIFIC PUBLISHERS LTD, IE, vol. 112, no. 2, 19 May 2007 (2007-05-19), pages 248-254, XP022085297, ISSN: 0378-8741, DOI: 10.1016/J.JEP.2007.03.005**

**(Cont. next page)**

- **ANALES DE LA REAL ACADEMIA DE FARMACIA VALLADARES J; PALMA M; SANDOVAL C; CARVAJAL F: "CREAM WITH OIL FROM MOSQUETA ROSA-RUBIGINOSA L II. STUDY OF PHYSICOCHEMICAL PROPERTIES STABILITY COSMETIC EFFICIENCY AND CLINICAL SYSTEMATIC APPLICATION", ANALES DE LA REAL ACADEMIA DE FARMACIA, vol. 52, no. 3, 1986, pages 597-612, XP009158771,**
- **MEDICINA CUTANEA IBERO-LATINO-AMERICANA CAMACHO F; MORENO J C; CONEJO-MIR J S; BUENO J: "Treatment of scars and post-surgical defects with pure oil of rosa mosqueta seed Medicina Cutanea Ibero-Latino-Americana Camacho F; Moreno J C; Conejo-Mir J S; Bueno J", MEDICINA CUTANEA IBERO-LATINO-AMERICANA, vol. 22, no. 1, 1994, pages 23-30, XP009158767,**
- **THIELEMANN A M; ORREGO I; SANDOVAL M A; CHAVEZ H: "Determination of the efficacy of a cream containing Rosa Mosqueta oil for wrinkle attenuation Thielemann A M; Orrego I; Sandoval M A; Chavez H", ANALES DE LA REAL ACADEMIA DE FARMACIA, vol. 59, no. 2, 1993, pages 211-218, XP009158772,**
- **R. AMENTA ET AL: "Traditional medicine as a source of new therapeutic agents against psoriasis", FITOTERAPIA, vol. 71, 1 August 2000 (2000-08-01), pages S13-S20, XP055056160, ISSN: 0367-326X, DOI: 10.1016/S0367-326X(00)00172-6**
- **PIERI F A ET AL: "Antimicrobial profile screening of two oils of Copaifera genus", ARQUIVO BRASILEIRO DE MEDICINA VETERINARIA E ZOOTECNIA, vol. 64, no. 1, February 2012 (2012-02), pages 241-244, XP002693641, ISSN: 0102-0935**
- **KINNUNEN T ET AL: "ANTIBACTERIAL AND ANTIFUNGAL PROPERTIES OF PROPYLENE GLYCOL, HEXYLENE GLYCOL, AND 1,3-BUTYLENE GLYCOL IN VITRO", ACTA DERMATO-VENEREOLOGICA, TAYLOR & FRANCIS LTD, UNITED KINGDOM, vol. 71, no. 2, 1 January 1991 (1991-01-01), pages 148-150, XP000670892, ISSN: 0001-5555**
- **OLITZKY I: "Antimicrobial properties of a propylene glycol based topical therapeutic agent.", JOURNAL OF PHARMACEUTICAL SCIENCES MAY 1965, vol. 54, no. 5, May 1965 (1965-05), pages 787-788, XP002693642, ISSN: 0022-3549**

Remarks:
The file contains technical information submitted after the application was filed and not included in this specification

**Description**

**Field of the invention**

The present invention relates to compositions comprising an extract of *Chelidonium majus* and resin of *Copaifera officinalis* (*Copaiba*).

The invention also relates to the use of the composition in the field of medicine and cosmetics for the treatment of skin lesions characterised by epidermal hyperproliferation and dermal damage.

**Prior art**

The human skin is made up principally of three layers: a thinner upper layer, the epidermis, a thicker intermediate layer, the dermis, and a lower layer of subcutaneous tissue.

The keratinocytes are the most abundant cell type in the dermis (75-80% of the total number of cells in the human epidermis), whereas the dermis is principally made up of fibroblasts. Furthermore, the skin is well vascularised with a dense network of arterial and venous vessels composed of vascular endothelial cells.

The factors underlying maintenance of the homoeostasis of the skin, i.e. maintenance of normal tissue morphology, are correct regulation of proliferation, migration and viability, and of the cell metabolism in general, of the mitochondrial dehydrogenase activity in fibroblasts and keratinocytes, of the concentration of free radicals and of the oxidising and reducing cellular and extracellular environment, of the concentrations of intracellular and intercellular mediators such as cytokines, of the production of collagen by fibroblasts, and of the microbial colonisation of the skin.

Changes in the above-mentioned processes are known to be involved in various pathologies and conditions, including skin lesions characterised by epidermal hyperproliferation and dermal damage, possibly associated with microbial infections [Hernandez - Martinez, Repair, regeneration and fibrosis, in Pathology, ed. E. Rubin, J.L. Farber, Philadelphia, Lippincott, 1999; Satish L and Kathju S, Dermatology Research and Practice, 2010, Article ID 790234, 11 pages].

Consequently, every time a state of hyperproliferation of the epidermis occurs, associated with diseases and conditions characterised by a diminished regeneration and repair of the skin (dermal damage), and possibly associated with vascular lesions and/or associated with a microbial infection such as, for example, in acne in the active phase, in acne in the healing phase, in the presence of impure skin, in ageing of acneic skin, impure skins and skin ageing in general, in atopic dermatitis and in dermatitis in general, in the presence of psoriatic plaques, in skin dyschromias, in hyperseborrhoea, in pustules, in papules, in comedones, in skin fibrosis, in scar formation, in atrophic acneic scarring, in furuncles, in cracked heels, in the presence of ingrowing hairs, in onychocryptosis, in skin calluses, in verrucas, in cheratosis and the like, it is desirable to have the stimulation of the viability (revitalising effect) and/or proliferation and/or of the migration of fibroblasts and of vascular endothelial cells, and inhibition of the viability and/or of the proliferation of keratinocytes, a reduction in the concentration of free radicals (anti-radical and skin-protective effect), a reduction in the concentration of interleukin-1 beta (healing effect), antagonism against the inflammatory process and the oxidative damage, antagonism against the fibrotic process and possibly inhibition of the growth of microorganisms responsible for microbial infections of the skin.

*Chelidonium majus* (*C. majus*) is a perennial herbaceous plant which grows spontaneously in woodland in the Mediterranean region. It belongs to the genus *Chelidonium* and the *Papaveraceae* family. It grows to a height of 30-90 cm and is characterised by a yellow-orange latex which oxidises and darkens rapidly in air, which leaks from the stalks once they are broken [Bruni A. (1999), Farmacognosia generale e applicata [General and applied pharmacognosy, PICCINNUOVA LIBRARIA SpA Padova, pp. 300-301].

*Chelidonium majus* contains numerous bioactive substances, including compounds belonging to the following chemical classes: alkaloids, acids, lactones, carotenoids, vitamins, flavonoids, phytosteroids, saponins and proteins, which have been isolated and identified analytically by chromatographic methods. [Kim HK et al. Journal of Pharmaceutical Sciences, 1969, 58, 372-374; Slavik J and Slavikova L Collection of Czechoslovak Chemical Communications, 1977, 42, 2686-2693; Slavik J et al. Collection of Czechoslovak Chemical Communications, 1965, 30, 3697-3704; Tin-Wa M et al. Journal of Natural Products, 1972, 35, 87-89]. Isoquinolinic alkaloids are especially abundant in *C. majus.* In particular, *C. majus* is rich in protopine, coptisine, stylopine, berberine, chelidonine, sanguinarine and chelerythrine [Gu Y et al. Journal of Separation Science, 2010, 33, 1004-1009]. The use of *C. majus* as a keratolytic agent is known in folk medicine [Amenta R et al. Fitoterapia [Phytotherapy] Volume 71, Supplement 1, 2000, S13-S20]. The use is also known of some of its principal alkaloids as keratolytic agents [WO09112226; US20060045930].

However, said preparations of *C. majus* or of alkaloids of *C. majus* have no dermal regeneration or dermal repair-stimulating effects, as well as no angiogenesis-stimulating effects, no anti-fibrotic effects and no anti-radical effects. Furthermore alkaloids such as sanguinarine and chelerythrine have toxic and irritant effects on the skin in quantities commonly present in *C. majus* extracts.

WO2009/112226 describes pharmaceutical compositions comprising the alkaloids sanguinarine, chelerythrine and chelidonine, as an extract of *C. majus* for use in the treatment of warts, verrucas and psoriatic plaques.

DE4031960 relates to a pharmaceutical composition comprising *C.majus* for the treatment of psoriasis.

FR2661330 describes pharmaceutical and cosmetic compositions comprising *C.majus* for use in the treatment of corns, callouses and verrucas.

*Copaiba, Copaifera officinalis* resin, is defined as an oleoresin (*Copaiba* balsam) obtained from *Copaifera officinalis L.,* leguminosae family [CAS: 9000-12-8 / 8001-61-4. EINECS/ELINCS: 232-526-3 / 232-288-0]. *Copaiba is* composed almost exclusively of terpenes. Of these, the main one is beta-caryophylline, the proportion of which in the oleoresin varies from approximately 20% to approximately 60%. *Copaiba* is known above all for its anti-inflammatory effect and anti-microbial action, as well as for its use in folk medicine for the treatment of skin lesions in general [Veiga Junior VF et al. Journal of Ethnopharmacology, 2007, 112, 248-254; Oliveira dos Santos A et al. Mem Inst Oswaldo Cruz, Rio de Janeiro, 2008, 103, 277-281].

*Rosa mosqueta* oil is obtained from seeds of the *Rosa Aff. rubiginosa,* a native of the South American Andes known locally as "*Rosa mosqueta*". The oil contains a large quantity of polyunsaturated fatty acids, approximately 70-80%, including linoleic acid derivatives in a quantity of 40-60%. In folk medicine, the *Rosa mosqueta* oil is used for the treatment of skin lesions in general.

Silicone compounds, in particular dimethicone, are widely used in dermatological preparations as emollient and protective agents for the skin.

With the aim of extending the possibility of treating these diseases and conditions, there is still a need for a composition which is effective in treating and preventing diseases and conditions of the skin which require stimulation of the regeneration and repair of the skin associated with inhibition of epidermal hyperproliferation and possibly stimulation of angiogenesis. In particular, a new composition is needed that is capable of determining stimulation of the viability and migration of fibroblasts and of vascular endothelial cells, and inhibition of the viability of keratinocytes, and which also has antifibrotic, wound healing, cytoprotective and anti-radical effects, and possibly also antimicrobial activity.

The aim of the present invention is therefore to provide particular compositions of *Chelidonium majus* and *Copaifera officinalis* (*Copaiba*) resin that are clinically safe and usable for treating various disorders of the skin, such as acne, impure skin, skin ageing, dermatitis, psoriasis, skin dyschromias, skin fibrosis, scarring and hyperseborrhoea, and which do not the above-mentioned disadvantages of the known compositions for the same application.

## Summary of the invention

The aim indicated above has been achieved with compositions comprising a *Chelidonium majus* extract and *Copaiba.*

Surprisingly, it was found that the formulations of the invention show one or more of following effects:

- scavenger activity versus free radicals,
- modulation of the mitochondrial dehydrogenase activity,
- modulation of collagen production,
- modulation of the production of IL-1$\beta$,
- modulation of the production of IL-6,
- modulation of cell migration,
- antimicrobial activity,
- absence of irritant effects.

The above effects indicate that the related formulations are well suited for uses in the medical and cosmetic fields described hereinafter.

## Uses in the medical and cosmetic fields

Anti-radical (skin-protective): this is deduced from the scavenger effects of free radicals.

Revitalising: this is deduced from the effect of increasing the mitochondrial dehydrogenase activity.

Anti-fibrotic (against scarring in general, against atrophic scarring, against hypertrophic scarring, against acneic scarring): this is deduced from an antagonistic effect on collagen stimulation induced by 34 ng/ml TGF-$\beta$1 and from an antagonistic effect on the stimulation of IL-1$\beta$ and/or of IL-6 induced by 34 ng/ml TGF-$\beta$1, and from inhibition of the production of IL-1$\beta$ and/or of IL-6 under standard growth conditions.

Cytoprotective against oxidative damage (antioxidant, anti-inflammatory and anti-ageing): this is deduced from an antagonistic effect on the stimulation of IL-1$\beta$ and/or of IL-6 induced by 100 $\mu$M $H_2O_2$ and the absence of changes in cell morphology which refer to cell senescence.

Wound healing: this is deduced from an inhibitory effect on the production of IL-1β and from a stimulating effect on cell proliferation under standard growth conditions. Anti-microbial: this is deduced from an inhibitory action on the growth of microorganisms such as bacteria, yeasts and/or fungi.

**Toxic effects**

Cytotoxicity: this is deduced from an effect of reducing mitochondrial dehydrogenase activity, or observation of changes in cell morphology which refer to cell senescence.

Irritation: this is deduced from an effect of increasing the production of IL-6 and/or of IL-1β under standard growth conditions.

Therefore, for the formulations of the invention, properties and characteristics described below were surprisingly found

Anti-radical

The formulations (4 and 8) show anti-radical effects.

Collagen

The formulations (4 and 8) at the concentration of 0.1% under standard growth conditions show a tendency to inhibit the production of collagens. They also antagonise the stimulation of collagen induced by 34 ng/ml TGF-β1. Conversely, in the presence of 100 μM $H_2O_2$, they have a tendency to antagonise the reduction in collagen induced by $H_2O_2$.

At the concentration of 0.1% w/v, the formulations cause inhibition of the production of collagen associated with anti-radical effects (scavenger activity) which also tend to counteract the oxidising action of $H_2O_2$ and consequently its effects on collagen production.

IL-1β

The formulations (4 and 8) at the concentration of 0.1% under standard conditions inhibit the production of IL-1β. They also antagonise the production of IL-1β induced by 100 μM $H_2O_2$ or by 34 ng/ml TGF-β1.

IL-6

The formulations (4 and 8) at the concentration of 0.1% under standard growth conditions do not significantly influence of production of IL-6. They do not show any significant effects on the production of IL-6 induced by 100 μM $H_2O_2$. However, they do antagonise production of IL-6 induced by 34 ng/ml TGF-β1.

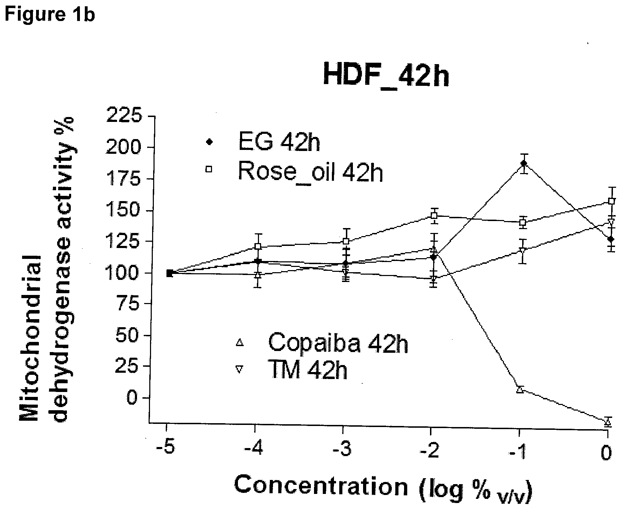

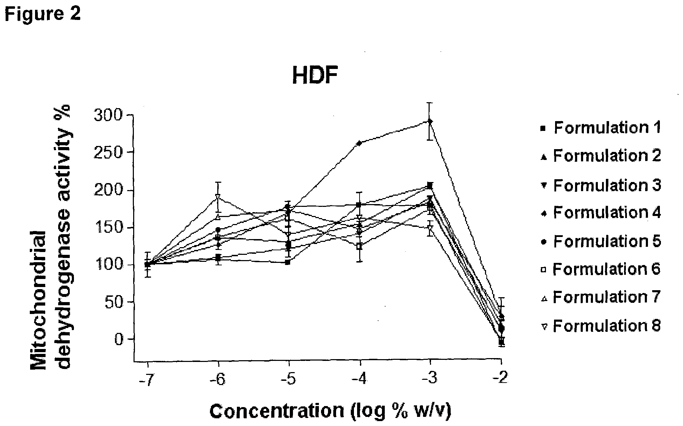

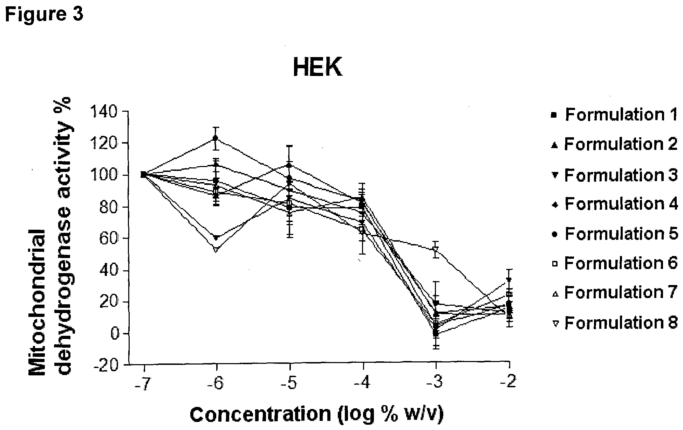

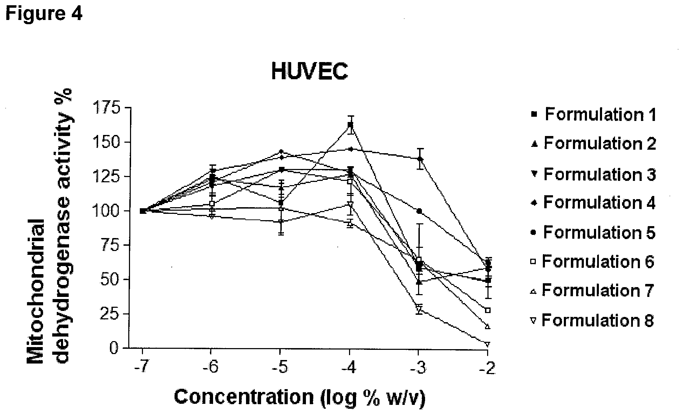

It has also surprisingly been found that the compositions of the invention, containing a *Chelidonium majus* extract and *Copaiba,* show a stimulating effect on the mitochondrial dehydrogenase activity and the migration of fibroblasts and vascular endothelial cells (see Figs. 2 and 4, respectively), but they show an inhibitory effect on the mitochondrial dehydrogenase activity of keratinocytes (see Figure 3). This effect is surprising in that, although the *Chelidonium majus* extracts stimulate the mitochondrial dehydrogenase activity of fibroblasts at low concentrations, and *Copaiba* at the same concentrations markedly inhibits the mitochondrial dehydrogenase activity of said cells (see Figure 1b), when these two substances are used in a formulation in equal quantities by weight, we observe a marked stimulation of the mitochondrial dehydrogenase activity of fibroblasts (see Figure 2). This means that *Copaiba* does not antagonise the stimulating effect of *C. majus* on the mitochondrial dehydrogenase activity: on the contrary, it potentiates such an effect. In other words, combining *Copaiba* with *C. majus* extract in a formulation results in a potentiation of the fibroblast mitochondrial dehydrogenase activity-stimulating effects if compared with the individual substances.

It has also been found that propylene glycol, used as an ingredient in certain preferred formulations, shows antimicrobial activity against various microorganisms, bacteria, fungi and yeasts.

Therefore the results of this research led the authors to the conclusions reported below and to the identification of uses in the medical and cosmetic fields for the formulations of the invention:

the formulations do not present toxic (cytotoxic and irritant) effects.

Furthermore, it was surprisingly found that the formulations of the invention can be used as anti-radical agents (for example as skin protectors), skin revitalising agents, antifibrotic agents (for example as prophylactics of scar formation, including acne scars), cytoprotective agents (for example as antioxidants, anti-inflammatory and anti-ageing agents, particularly useful for the treatment of acne), wound healing agents, and possibly antimicrobial agents.

Under another aspect, the invention relates to a composition comprising *Chelidonium majus* extract and *Copaiba,* wherein said composition comprises berberine and beta-caryophyllene, wherein the ratio between the amount in weight of beta-caryophyllene and the amount in weight of berberine is within the range from 7,000 to 60,000,000.

Under a further another aspect, the invention relates to a composition wherein said *Chelidonium majus* extract comprises protopine, stylopine, chelidonine, sanguinarine, berberine, chelerythrine and coptisine and wherein:

- the ratio between the amount in weight of stylopine and the total amount in weight of sanguinarine, chelerythrine, chelidonine and protopine is greater than or equal to 5;
- the ratio between the amount in weight of berberine and the total amount in weight of sanguinarine, chelerythrine, chelidonine, protopine is greater than or equal to 1; and
- the ratio between the amount in weight of berberine and the amount in weight of coptisine in the *Chelidonium majus* extract is greater than or equal to 0.05.

Under another aspect, the invention relates to a composition comprising a *Chelidonium majus* extract and *Copaiba* and *Rosa mosqueta* oil.

Indeed, it was also surprisingly found that the *Rosa mosqueta* oil further potentiates the fibroblast mitochondrial dehydrogenase activity-stimulating effects of the composition comprising *Copaiba* and *Chelidonium majus* extract (see Figure 2, examples 3, 4, 7 and 8).

Under another aspect, the invention relates to a composition comprising a *Chelidonium majus* extract and *Copaiba* and a silicone a silicone compound, preferably dimethicone.

Indeed, it was surprisingly found that the presence of a silicone compound in a composition does not alter the mitochondrial dehydrogenase activity-modulating effects of the composition comprising *Chelidonium majus* extract and *Copaiba* and *Rosa mosqueta* oil (see Figs. 2, 3 and 4, examples 1, 2, 3 and 4).

The compositions of the invention comprising a *Chelidonium majus* extract and *Copaiba,* and possibly containing *Rosa mosqueta* oil and a silicone compound, are preferably formulated in the form of a gel.

Indeed, it has also been surprisingly found that the formulation in gel form does not alter the mitochondrial dehydrogenase activity-modulating effects of the composition comprising *Chelidonium majus* extract and *Copaiba* and *Rosa mosqueta* oil (see Figs. 2, 3 and 4).

According to the above, it can be deduced that such modulation of the mitochondrial dehydrogenase activity determined by a composition of the invention will have a beneficial effect on the treatment and in the prevention of diseases and conditions of the skin which require stimulation of the regeneration and repair of the dermis associated with inhibition of epidermal hyperproliferation and possibly stimulation of angiogenesis.

Furthermore, in consideration of the scavenger activity on free radicals, the mitochondrial dehydrogenase activity-modulating effects, the IL-1$\beta$ production-modulating effects, the antagonistic effects on the release of interleukins IL-6 and IL-1$\beta$ induced by TGF-$\beta$1 or by $H_2O_2$, the antagonistic effects on the production of collagen induced by TGF-$\beta$1 and the antimicrobial activity of propylene glycol, the authors surprisingly found that the formulations of the invention can be used as anti-radical agents (for example as skin protectors), skin revitalising agents, antifibrotic agents (for example as prophylactics of scar formation, including acne scars), cytoprotective agents (for example as antioxidants, anti-inflammatories and anti-ageing agents, also useful for the treatment of acne), wound healing agents, and possibly antimicrobial agents.

In particular, the authors have found that, in view of the antimicrobial activity exerted by propylene glycol, the formulations of the invention which contain a glycolic extract of *C. majus* can be used as antimicrobial agents. In particular, in view of the ability of propylene glycol to inhibit the growth of *Propionibacterium acnes,* the formulations of the invention which contain a glycolic extract of *C. majus* can be used as anti-acne agents.

Therefore, the use of formulations of the invention in the medical and cosmetic fields are summarised as follows:

- use as anti-radical agents with skin protection properties, in that they exert a scavenger action on the radical DDPH,
- use as skin revitalising agents, in that they are able to stimulate the mitochondrial dehydrogenase activity in dermal fibroblasts,
- use as antifibrotic agents and therefore as prophylactics in scar formation, including acne scars, in that they antagonise the production of collagen, IL-6 and IL-1$\beta$ stimulated by TGB-$\beta$1 in dermal fibroblasts,
- use as cytoprotective agents and therefore as antioxidants, anti-inflammatories and anti-ageing agents, also useful for the treatment of acne, in that they antagonise the production of IL-6 and IL-1$\beta$ stimulated by $H_2O_2$ in dermal fibroblasts,
- use as wound healing agents, in that they are able to reduce the production of IL-1$\beta$ in dermal fibroblasts,
- use as antimicrobial agents, including the case of treatment of acne, as for the preferred formulations of the invention which contain propylene glycol at a dosage capable of exerting an antimicrobial action against microorganisms such as *Escherichia coli, Pseudomonas aeruginosa, Bacteroides fragilis, Propionibacterium acnes, Candida albicans,*

*Candida parapsilosis, Aspergillus niger.*

Under yet another aspect, the invention relates to the composition of the invention for use in the treatment and prevention of skin lesions, of diseases and conditions of the skin, possibly associated with microbial infections, which require stimulation of dermal regeneration and repair associated with inhibition of epidermal hyperproliferation and possibly stimulation of angiogenesis, wherein said diseases and conditions of the skin are preferably selected from the group consisting of acne in its active phase, acne in its healing phase, the presence of impure skin, ageing of acneic skin, impure skins and skin ageing in general, atopic dermatitis and dermatitis in general, the presence of psoriatic plaques, skin dyschromias, hyperseborrhoea, pustules, papules, comedones, skin fibrosis, scar formation, atrophic acneic scarring, furuncles, cracked heels, the presence of ingrowing hairs, onychocryptosis, skin calluses, verrucas, cheratosis and the like.

**Brief description of the drawings**

The characteristics and advantages of the present invention will be obvious from the detailed description given below, and from the example embodiments provided by way of non-limiting illustration, as well as by the attached drawings, wherein:

Figure 1 shows the effect of *C. majus* extracts, *Copaiba* and *Rosa mosqueta* oil on the mitochondrial dehydrogenase activity of human dermal fibroblasts (HDF). In the plots the mitochondrial dehydrogenase activity is reported on the y-axis as % versus the formulation concentrations (x-axis). In particular:

Figure 1a shows the plot of the dose/response curve at 6 concentrations of: glycolic extract of *C. majus* (EG), *Copaiba, Rosa mosqueta* oil (Olio_rosa), and mother tincture of *C. majus* (TM). The tests were performed after 18 hours with 8,000 cells seeded per well;
Figure 1b shows the plot of the dose/response curve at 6 concentrations of: glycolic extract of *C. majus* (EG), *Copaiba, Rosa mosqueta* oil (Olio_rosa), and mother tincture of *C. majus* (TM). The readings were performed after 42 hours with 2,000 cells seeded per well;

Figure 2 shows the effect of eight formulations on the mitochondrial dehydrogenase activity of human dermal fibroblasts (HDF). Dose/response curves were plotted at 6 concentrations. The readings were performed after 42 hours with 2,000 cells seeded per well. In the plots, the mitochondrial dehydrogenase activity in % is given on the y-axis, versus the formulation concentrations given on the x-axis.
Figure 3 shows the effect of eight formulations of the invention on the mitochondrial dehydrogenase activity of human epidermal keratinocytes (HEK). In particular, the dose/response curves are shown at 6 concentrations. The readings were performed after 24 hours with 8,000 cells seeded per well.
Figure 4 shows the eight formulations of the invention on the mitochondrial dehydrogenase activity on human umbilical vascular endothelial cells (HUVEC). In particular, the dose/response curve are shown at 6 concentrations. The readings were performed after 24 hours with 8,000 cells seeded per well.
Figure 5 shows photomicrographic images at various magnifications of HDF with Sirius Red collagen staining and subjected to various treatments with formulations 4 and 8 at the specified concentrations, for 24 hours, under standard growth conditions.
Figure 6 shows photomicrographic images at various magnifications of HDF with Sirius Red collagen staining and subjected to various treatments with formulations 4 and 8 at the specified concentrations, for-24 hours, in the presence of 100 $\mu$M $H_2O_2$.
Figure 7 shows photomicrographic images at various magnifications of HDF with Sirius Red collagen staining and subjected to various treatments with formulations 4 and 8 at the specified concentrations, for 24 hours, in the presence of 34 ng/ml TGF-$\beta$1.

**Detailed description of the invention**

The invention thus relates to a composition comprising a *Chelidonium majus* extract and *Copaiba.*
As the compositions are aimed at use in the cosmetic and medical-pharmaceutical fields, such compositions and such extracts must be clinically safe and well suited for use to treat various infections of the skin.
An object of the present invention is a composition comprising a *Chelidonium majus* extract and *Copaiba.*
In a preferred embodiment, the invention relates to a composition comprising a *Chelidonium majus* extract and *Copaiba.* Said composition comprises berberine and beta-caryophyllene, wherein the ratio between the amount in weight of beta-caryophyllene and the amount in weight of berberine ranges from 7,000 to 60,000,000.

For the purposes of the present invention:

- "*Chelidonium majus*" or "*C. majus*" or "Celandine" mean a perennial herbaceous plant of the Mediterranean region which belongs to the genus *Chelidonium* and the *Papaveraceae* family and is characterised by a yellow-orange latex, which leaks from the stalks once they are broken and oxidises rapidly (darkens) in air. The invention is intended to comprise all the varieties and sub-species of the plant, such as *Chelidonium majus* var. *majus* e *Chelidonium majus* var. *laciniatum;*
- "Aerial parts of the *Chelidonium majus* plant" mean organs of the plant which, under natural conditions, developed above ground, such as flowers, buds and leaves;
- "*Chelidonium majus* extract" means a liquid preparation (fluid extraction), solid preparation (dry extraction) or preparation of intermediate consistency (soft extraction) obtained by process of extraction from the whole of or part of the *Chelidonium majus* plant, in the form or fresh or air-dried or freeze-dried plant or after being subjected to any other stabilisation or preservation procedure.
- "Copaiba" means the resin of *Copaifera officinalis,* an oleoresin (balsam of *Copaiba*) obtained from *Copaifera officinalis L.,* Leguminosae; and
- "*Rosa mosqueta* oil" means an oil obtained from the seeds of *Rosa Aff. rubiginosa,* native to the South American Andes.

In a further preferred embodiment, the composition according to the invention comprises a *Chelidonium majus* extract and *Copaiba,* wherein said *Chelidonium majus* extract comprises protopine, stylopine, chelidonine, sanguinarine, berberine, chelerythrine and coptisine and wherein:

- the ratio between the amount in weight of stylopine and the total amount in weight of sanguinarine, chelerythrine, chelidonine and protopine is greater than or equal to 5;
- the ratio between the amount in weight of berberine and the total amount in weight of sanguinarine, chelerythrine, chelidonine, protopine is greater than or equal to 1; and
- the ratio between the amount in weight of berberine and the amount in weight of coptisine in the *Chelidonium majus* extract is greater than or equal to 0.05.

In a further preferred embodiment, the composition according to the present invention further comprises *Rosa mosqueta* oil.
Indeed, it was also surprisingly found that the *Rosa mosqueta* oil further potentiates the fibroblast mitochondria dehydrogenase activity-stimulating effects of the composition comprising *Copaiba* and *Chelidonium majus* extract (see Figure 2, examples 3, 4, 7 and 8).
Under yet another aspect, the invention relates to a composition comprising a *Chelidonium majus* extract and *Copaiba* and propylene glycol.
Under yet another aspect, the invention relates to a composition comprising a *Chelidonium majus* extract and *Copaiba* and a silicone compound, preferably dimethicone.
Indeed, it was surprisingly found that the presence of a silicone in the composition does not alter the mitochondrial dehydrogenase activity-modulating effects of the composition comprising *Chelidonium majus* extract and *Copaiba* and *Rosa mosqueta* oil (see Figs. 2, 3 and 4, examples 1, 2, 3 and 4).
In another preferred embodiment, the invention relates to a composition wherein the ratio between the amount in weight of beta-caryophyllene and the amount in weight of silicone compound is within the range from 10 to 40.
Advantageously, the composition according to the invention comprises approximately 10% *Chelidonium majus* extract and approximately 10% *Copaifera officinalis,* referred to the total of the composition.
In a preferred form, the invention relates to pay composition further comprising an amount of *Rosa mosqueta* oil ranging from 5 to 10% and an amount of dimethicone ranging from 0 to 0.15%, referred to the total of the composition. Under another aspect, the present invention relates to the composition according to the invention for use in the treatment and prevention of skin lesions characterised by epidermal hyperproliferation and dermal damage.
The term "skin lesion characterised by epidermal hyperproliferation and dermal damage" means states in which keratinocytic hyperproliferation of the epidermis occurs in associaton with dermal damage characterised by reduced regeneration and repair of the dermis itself, i.e. states such as acne in the active phase, acne in the healing phase, the presence of impure skin, ageing of acneic skin, impure skins and skin ageing in general, atopic dermatitis and dermatitis in general, the presence of psoriatic plaques, skin dyschromias, hyperseborrhoea, pustules, papules, comedones, skin fibrosis, scar formation, atrophic acneic scarring, furuncles, cracked heels, the presence of ingrowing hairs, onychocryptosis, skin calluses, verrucas, cheratosis and the like.
Under another aspect, the invention relates to the composition of the present invention for use in the cosmetic field.

The cosmetic compositions comprise a *Chelidonium majus* extract and *Copaiba* according to the invention and a cosmetically acceptable excipient.

In certain embodiments, the cosmetically acceptable vehicle of the composition of the invention is an excipient, vehicle or diluent suitable for the pharmaceutical formulations.

The compositions may be formulated, for example in gels, lotions, foams, sticks, ointments, milks or creams.

In certain embodiments, the composition according to the invention is applied to the skin in need of treatment.

In certain embodiments, the composition is in the medical device, or in a medicinal pharmaceutical form for topical use.

Under yet another aspect, the invention relates to a composition comprising a *Chelidonium majus* extract and *Copaiba,* for use in the treatment of skin lesions characterised by epidermal hyperproliferation and dermal damage.

In a preferred form, said skin lesions characterised by epidermal hyperproliferation and dermal damage are selected from the group consisting of acne, impure skin, skin ageing, dermatitis, psoriasis, skin dyschromia, keratosis and hyperseborrhoea.

In a yet more preferred form, skin lesions characterised by epidermal hyperproliferation and dermal damage are selected from the group consisting of acne and keratosis.

The *Chelidonium majus* extracts are prepared by a particular procedure which may advantageously be a procedure of extraction (for example extraction by maceration, extraction by infusion, extraction by digestion, or extraction by percolation) and may be performed under various conditions of temperature, pressure and extraction time.

Said *Chelidonium majus* extract is preferably an extract obtained from the fresh plant in propylene glycol. Advantageously, in said extract the ratio between the plant and the solvent is 20% w/v and is performed at room temperature for 28 days. The extract is recovered by settling the solvent, pressing the substance, and filtering the whole mixture.

More preferably, said extract is obtained from the aerial parts of the *Chelidonium majus* plant, selected from the group consisting of flowers, buds and leaves, preferably leaves without the veins.

In the following, example embodiments of the present invention are provided by way of illustration.

## EXAMPLES

### Example 1: PREPARATION AND CHEMICAL CHARACTERISATION OF C. MAJUS EXTRACTS, OLEORESIN OF *COPAIBA,* AND OIL OF *ROSA MOSQUETA*

### Preparation of the glycolic extract of aerial parts of *C. Majus* (EG)

The aerial parts of the plant, collected in the Monti Ernici (Rome, Italy) were minced and placed in a pot in the presence of propylene glycol in a ratio of 20% w/v between the plant and the solvent, at room temperature for 28 days. After this time, the extract was recovered by settling the solvent, also pressing the whole material and filtering the liquid product obtained in order to remove any remaining herb particles.

### Preparation of the mother tincture of *C. majus* (TM)

The aerial parts of the plant, collected in the Monti Ernici (Rome, Italy) were minced and placed in a pot in the presence of alcohol at 45°C in a ratio between the plant and the solvent of 33% w/v, at room temperature for 28 days. After this time, the extract was recovered by settling the solvent, also pressing the whole material and filtering the liquid product obtained in order to remove any remaining herb particles.

### Preparation of the oleoresin of *Copaiba*

The oleoresin of *Copaifera officinalis* or *Copaiba* was provided by Beraca (San Paolo, Brazil). The preparation procedure, as described by the supplier, is reported below: two holes are made in the trunk of the *Copaiba* tree, one at a height of 60-90 cm, and the other 3-6 m higher up. A tubular piece of bamboo, endowed with a simple stopper, is fitted into the lowest hole. Under normal conditions, the *Copaiba* flows easily and is collected in tanks. After the operation is completed, both holes are closed with simple stoppers, the tree keeps growing, enabling other future tappings of *Copaiba* oil.

### Preparation of the oil of *Rosa mosqueta*

The *Rosa mosqueta* oil was supplied by Agrar srl (Rome, Italy). The preparation procedure, as described by the supplier, is reported below. It was prepared by cold-pressing the seeds of *Rosa aeruginosa* originating from South America, refined, neutralised and stabilised with natural vitamin E at a concentration of 300 ppm.

The above analysis was performed by HPLC in accordance with a modification of the procedure described in Sarkozi A et al. Chromatographia, 2006, 63, pp. 81-86. This modification allowed the alkaloid stylopine to be also identified.

In detail, the analyses were performed on an HPLC Perkin-Elmer Flexar composed of a binary pump, a three-channel degasser, and a UV/VIS detector. The programme TotalChrome was used for data acquisition and processing.

The separation was performed on a Phenomenex Luna 5 $\mu$l C18 reversed-phase column (250 mm x 4.6 mm l.D.). The wavelength used was 280 nm. The injection volume was 20 $\mu$l (loop measurement). An isocratic mixture of acetonitrile-methanol-ammonium formiate 30 mM (pH 2.8) 150:180:670 was used as the eluent. The flow rate was 0.8 ml/min. The identification of the components was made by adding suitable samples of "standards", i.e. known individual components. The amount of each "standard", added to 35 $\mu$l of sample to be anlyzed, was 5 $\mu$l.

The *C. majus* extracts are characterised by the concentrations of the main alkaloid. Such a composition is reported in Table 1 (see below), where the concentrations are expressed in ppm.

**Table 1.**

| Extract | Protopine | Chelidonine | Coptisine | Stylopine | Sanguinarine | Berberine | Chelerythrine |
|---|---|---|---|---|---|---|---|
| Glycolic extract (EG) | 7.49 | 12.96 | 239.24 | 112.60 | 0.08 | 21.63 | <0.01 |
| Mother tincture (TM) | 0.21 | 29.29 | 18.89 | 0.34 | <0.01 | 0.01 | <0.01 |

The concentration of berberine may change, ranging however from 0.01 to 22 ppm (mg/l).

## Chemical analysis of *Copaiba*

The oleoresin of *Copaiba* is a viscous, translucent liquid, of a transparent greenish colour. It is characterised by following physicochemical parameters:

Specific weight: from 0.850-0.950 g/cm$^3$, acidity value: <= 2.0, peroxide value: <= 10.0 and percentage of $\beta$-caryophyllene: (GLC) >= 42%.

Since the minimum density of 0.85, 1 l of copaiba weighs 850 g, and therefore it contains an amount of beta-caryophyllene greater than or equal to 850*42% = 357 g, or a concentration of 357.000 ppm. Given that the concentration of beta-caryophyllene in various commercially available oils of *Copaiba* ranges from about 20 to about 60% and that the density of *Copaiba* ranges between 0.85 and 0.95, according to an analogous calculation, the concentration of beta-caryophyllene in *Copaiba* can range between 1*0.85*0.2*1000*1000=170.000 ppm and 1*0.95*0.6*1000*1000= 570.000 ppm.

## Chemical analysis of the oil of *Rosa mosqueta*

The *Rosa mosqueta* oil is characterised by the physicochemical parameters given in Table 2 (see below).

**Table 2.** Physicochemical parameters characterising the *Rosa mosqueta* oil

| |
|---|
| - INCI name (new as = 2006): musk rose seed oil |
| - Description reddish-yellow clear liquid with a slight characteristic odour |
| - Origin: from the seeds of the plant; subsequently refined, neutralised and stabilised with natural vitamin E (300 ppm) |
| - Specific gravity at 20°C: 0.925 g/ml |
| - Refraction index: 1.478 |
| - Colour (Gardner): conforms |
| - Colour (lovibond): 26 Y / 2.4 R |
| -Humidity (K. F.): 0.03% |
| - Iodine value (Wijs): 169 g/100g |
| - Saponification value: 191 mgKOH/g |
| - Acid value: 0.10 mgKOH/g |
| - Peroxide value: 1.4 meqO2/g |
| - Unsaponifiables: approx. 0.5-2.5% |
| -Viscosity at 25°Capprox. 40-50 cps |

(continued)

| COMPOSITION of the main FATTY ACIDS (mean): |
| --- |
| - C-16:0 - Palmitic: 2.5-5.0% |
| - C-16:1 - Palmitoleic: 0.1-0.4% |
| - C-17.0 -Heptadecanoic: 0.1-0.2% |
| - C-18:0 - Stearic: 1.0-5.0% |
| - C-18:1 - Oleic: 13.0-19.0% |
| - C-18:2 - Linoleic: 42.0-55.0% |
| - C-18:3 - Linolenic: 27.0-40.0% |
| - C-20:0 - Arachic: 0.3-2.0% |
| Total polyunsaturated fatty acids: 77-80% |

The concentration of linoleic acid is within the limits of 1*0.925*0.42*1000*1000= 388.000 and 1*0.925*0.55*1000*1000= 509.000, expressed as ppm.

**Example 2: Formulations**

The following formulations were prepared:

| Formulation | *Chelidonium majus* extract present | Presence of *Copaiba* | Presence of *Rosa mosqueta oil* | Presence of dimethicone |
| --- | --- | --- | --- | --- |
| 1 | Mother tincture | Yes | No | Yes |
| 2 | Glycolic extract | Yes | No | Yes |
| 3 | Mother tincture | Yes | Yes | Yes |
| 4 | Glycolic extract | Yes | Yes | Yes |
| 5 | Mother tincture | Yes | No | No |
| 6 | Glycolic extract | Yes | No | No |
| 7 | Mother tincture | Yes | Yes | No |
| 8 | Glycolic extract | Yes | Yes | No |

Formulation 1: Cosmetic gel containing *Chelidonium majus, Copaifera officinalis* and a silicone compound

| | |
| --- | --- |
| Water | q.s. ad 100 |
| *Chelidonium majus* (mother tincture) | 10 |
| *Copaifera officinalis* resin | 10 |
| Dimethicone | 0.15 |
| Carbomer | 0.6 |
| Phenoxyethanol | 0.50 |
| Sodium methylparaben | 0.20 |
| Sodium propylparaben | 0.15 |

Formulation 2: Cosmetic gel containing *Chelidonium majus, Copaifera officinalis* and a silicone compound

| | |
| --- | --- |
| Water | q.s. ad 100 |
| *Chelidonium majus* (glycolic extract) | 10 |
| *Copaifera officinalis* resin | 10 |

(continued)

| | |
|---|---|
| Dimethicone | 0.15 |
| Carbomer | 0.6 |
| Phenoxyethanol | 0.50 |
| Sodium methylparaben | 0.20 |
| Sodium propylparaben | 0.15 |

Formulation 3: Cosmetic gel containing *Chelidonium majus, Copaifera officinalis, Rosa mosqueta* and a silicone compound

| | |
|---|---|
| Water | q.s. ad 100 |
| *Chelidonium majus* (mother tincture) | 10 |
| *Copaifera officinalis* resin | 10 |
| *Rosa mosqueta* oil | 5 |
| Dimethicone | 0.15 |
| Carbomer | 0.6 |
| Phenoxyethanol | 0.50 |
| Sodium methylparaben | 0.20 |
| Sodium propylparaben | 0.15 |

Formulation 4: Cosmetic gel containing *Chelidonium majus, Copaifera officinalis, Rosa mosqueta* and a silicone compound

| | |
|---|---|
| Water | q.s. ad 100 |
| *Chelidonium majus* (glycolic extract) | 10 |
| *Copaifera officinalis* resin | 10 |
| *Rosa mosqueta* oil | 5 |
| Dimethicone | 0.15 |
| Carbomer | 0.6 |
| Phenoxyethanol | 0.50 |
| Sodium methylparaben | 0.20 |
| Sodium propylparaben | 0.15 |

Formulation 5: Cosmetic gel containing *Chelidonium majus* and *Copaifera officinalis*

| | |
|---|---|
| Water | q.s. ad 100 |
| *Chelidonium majus* (mother tincture) | 10 |
| *Copaifera officinalis* resin | 10 |
| Carbomer | 0.6 |
| Phenoxyethanol | 0.50 |
| Sodium methylparaben | 0.20 |
| Sodium propylparaben | 0.15 |

Formulation 6: Cosmetic gel containing *Chelidonium majus* and *Copaifera officinalis*

| Water | q.s. ad 100 |
|---|---|
| *Chelidonium majus* (glycolic extract as) | 10 |
| *Copaifera officinalis* resin | 10 |
| Carbomer | 0.6 |
| Phenoxyethanol | 0.50 |
| Sodium methylparaben | 0.20 |
| Sodium propylparaben | 0.15 |

Formulation 7: Cosmetic gel containing *Chelidonium majus, Copaifera officinalis* and *Rosa mosqueta*

| Water | q.s. ad 100 |
|---|---|
| *Chelidonium majus* (mother tincture) | 10 |
| *Copaifera officinalis* resin | 10 |
| *Rosa mosqueta* oil | 10 |
| Carbomer | 0.6 |
| Phenoxyethanol | 0.50 |
| Sodium methylparaben | 0.20 |
| Sodium propylparaben | 0.15 |

Formulation 8: Cosmetic gel containing *Chelidonium majus, Copaifera officinalis* and *Rosa mosqueta*

| Water | q.s. ad 100 |
|---|---|
| *Chelidonium majus* (glycolic extract) | 10 |
| *Copaifera officinalis* resin | 10 |
| *Rosa mosqueta* oil | 5 |
| Carbomer | 0.6 |
| Phenoxyethanol | 0.50 |
| Sodium methylparaben | 0.20 |
| Sodium propylparaben | 0.15 |

**EXAMPLE 3: BIOASSAYS AND TESTS FOR ANTI-RADICAL ACTIVITY**

Cell cultures and treatments

The human dermal fibroblasts (HDF) were grown and handled according to the supplier's instructions (ECACC, cat. 106-05a, http://www.hpacultures.org.uk/). The human keratinocytes (HEK, Human Epidermal Keratinocyte, ECACC) were grown and handled according to the supplier's instructions (ECACC, cat. 102-05a, http://www.hpacultures.org.uk/).
The HUVEC cells (Human Umbilical Vein Endothelial Cells, ECACC) were grown and handled according to the supplier's instructions (ECACC, cat. 200-05n, http://www.hpacultures.org.uk/).
For what concerns the quantification of inflammatory cytokines in the cell supernatant, analysed using the ELISA method, the quantification of collagen produced by the HDF cells, evaluation of the cell proliferation, and evaluation of cell morphology, the cells were seeded into 24-well flat-bottom plates (Corning), allowed to adhere and then treated in triplicate or with the test formulation at a given concentration, or with control media in the presence or absence of 100 $\mu$M $H_2O_2$ or of 34 ng/ml TGF-$\beta$1. The conditions under which the cells underwent the treatments in triplicate, either with the test formulation at the desired concentration or with control media, are defined as "standard growth conditions". The total volume of culture medium per well was 500 $\mu$l. After a 24-hour period of treatment, the cell supernatant or the cells were analysed as better described in paragraphs "Measurement of inflammatory cytokines in the cell supernatant" and "Fixation of the cells will be methanol".
In respect of the tests aimed at evaluating mitochondrial dehydrogenase activity, HDF, HEK and HUVEC cells were seeded into 96-well flat-bottom plates (Sarstedt), at a density from 2,000 a 8,000 cells per well, allowed to adhere and then treated in quadruplicate either with the test formulation at the desired concentration or with control medium. The total volume of culture medium per well was 100 $\mu$l. The test formulations were evaluated at concentrations ranging from 1%v/v to 0.000001%v/v in the case of individual components. Human dermal fibroblasts were used for testing at T75 passage III. Human keratinocytes were used for testing between passage I and passage VI. HUVEC cells were used for testing between passage I and passage VI.

Following a period of treatment of 18 hours or 42 hours, the cells were analysed as better described in the paragraph "Evaluation of the mitochondrial dehydrogenase activity".

In respect of the tests aimed at evaluating cell migration, the culturing of fibroblasts or HUVEC cells was performed on 96-well flat-bottom plates (Sarstedt), bringing the cells to confluence. The evaluation of the effects of the formulations, at a concentration of 0.02% v/v, on the migration of fibroblasts or HUVEC cells was performed as described in the paragraph "Evaluation of cell migration modulation".

Evaluation of the mitochondrial dehydrogenase activity

The mitochondrial dehydrogenase activity was measured in HDF, HEK and HUVEC by means of the formazan test, using the Roche Diagnostic WST-1 kit in accordance with the supplier's instructions. Briefly, the reactant was added (10 $\mu$l per well), the incubation was continued for another 2 hours or 4 hours, and at the end of this time the absorbance was read for 1 sec at a wavelength of 450 nm-in a Perkin Elmer Victor 2 Wallac microplate reader.

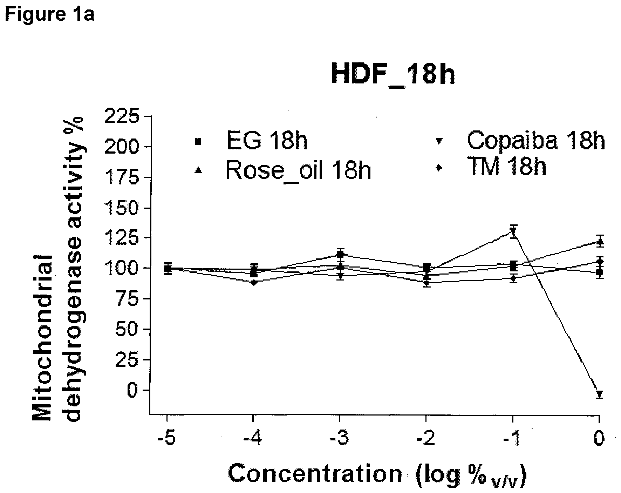

The results are given in Figures 1 to 4, wherein mitochondrial dehydrogenase activity expressed as activity percentage (mitochondrial dehydrogenase activity %) is defined as: Mitochondrial dehydrogenase activity % = ((absorbance of the treated cells - absorbance of the blank) / (absorbance of the control - absorbance of the blank)) x 100.

Measurement of inflammatory cytokines in cell supernatant

Qualitative and quantitative determinations were performed of the inflammatory cytokines, CD40 ligand (CD40L), interferon-$\gamma$ (INF-y), interleukin-1$\alpha$ (IL-1$\alpha$), interleukin-1$\beta$ (IL-1$\beta$), interleukin-6 (IL-6), interleukin 8 (IL-8), interleukin-17 (IL-17) and tumor necrosis factor-a (TNF-$\alpha$). Each measurement was performed in duplicate using an R&D System Mosaic™ ELISA protein *microarray* in accordance with the supplier's instructions. Furthermore, with the aim of obtaining a model of correlation between the intensity of the light signal and the cytokines concentration, a calibration curve using 6 concentrations was obtained for each of the latter. Data analysis was performed using the Quansys Biosciences Q-View™ Imager dedicated software.

Fixation of the cells with methanol

After removing the cell supernatant, the HDF cells were fixed in cold methanol overnight at -20°C.

Protocol for staining the cells with Sirius Red for collagen

After fixing with methanol for one night, the solvent was removed and the fixed HDF cells were stained with 400 $\mu$l/well of a 0.1% solution of Picro-Sirius Red (PSR) stain for 4 hours. This solution is prepared by dissolving the reagent, Sigma-Aldrich Direct Red 80 in a saturated aqueous solution of picric acid. After 4 h incubation with PSR at room temperature, the cells were washed twice with 200 $\mu$l/well of a 0.1% aqueous solution of acetic acid, and dried.

Evaluation of cell proliferation

Photographs of the HDF cells stained with Sirius Red in all of the wells of the plates were taken at a magnification of 100x and their number, for each photograph was evaluated by means of the cell-counting function of the ImageJ program.

Evaluation of cell morphology

Moreover, Photographs of the HDF cells stained with Sirius Red in all of the wells of the plates were taken at 100x, 200x and 400x magnifications. Their morphology, colour and form were evaluated by means of the ImageJ program.

Measurement of the quantity of collagen produced by the cells by means of Sirius Red elution

The Sirius Red-stained HDF cells were subjected to elution of the stain in a way analogous to the one reported in "Am. J. Physiol. Renal. Physiol., 2007". Briefly, the stain retained within the cells was eluted with 200 $\mu$l/well of a 0.1 N aqueous solution of sodium hydroxide by shaking in an oscillating stirrer, followed by transferring samples of the eluted solution into 96-well plates. The optical density (OD) was read using a Wallac microplate reader in light absorbance at the wavelength 530 nm.

## Evaluation of the modulation of cell migration

This test was performed to evaluate the effects on fibroblasts migration of formulations at a concentration of 0.02% v/v.

Once at confluence, an artificial cross-shaped wound was made on the monolayer of cells, using a sterile plastic tip on each sample. The cell culture medium was removed and replaced by medium containing the test substance, with the exception of the negative controls.

Immediately after execution of the wound (t = 0 h) and at various times (t = 24 and 48 h) phase-contrast microscopy observations of the treated samples and of the controls were performed using an inverted phase-contrast microscope (INV-2 Orma) at a magnification going from 200X and 400X. The results are shown as the areas of the observation-contiguous to the wound, in which the presence of cells is observed. An increase in this area at increasing times corresponds to a reduction in the wound dimensions, an indication that the cells have migrated towards the area within the cut. This area was measured using the CellProfiler program [Carpenter AE et al. Genome Biology, 2006, 7, R100].

## Evaluation of anti-radical activity

In respect of the tests aimed at evaluating anti-radical activity, a chemical test was used in which the decay of the diphenylpicrylhydrazyl (DPPH) radical was evaluated in the absence or in the presence of test substances. In more detail, this radical is used to test the capacity of the substances to act as free radical scavengers. DPPH in solution has a purple colour, with a maximum absorption at 517 nm, which changes to yellow-colourless when this radical extracts a hydrogen atom from a radical scavenger, to produce the reduced form of DPPH-H. The test was performed on 96-well microplates (SARSTEDT Tissue Culture Plate 96-well flat bottom cell+) in quadruplicate. To each well of the microplate, 500 mg of formulation and then 5 $\mu$l of a 2 mM solution of DPPH were added (MW 168.18, Aldrich 2,2-di(4-tert-octylphenyl)-1-picrylhydrazyl, free radical Lot# MKBF7574V Cat N° 257621-100 mg).

A positive control, consisting of 500 mg of formulation with 5 mg of ascorbic acid, and a negative control, consisting of 500 mg of formulation with 5 $\mu$l of $H_2O_2$ at 36 volumes, were included in each microplate. 30 minutes after addition of DPPH, the colours of the controls and of the formulation were evaluated, and a photograph of the plate (Canon PowerShot A610 5.0 Mpixels) was taken; then the potential anti-radical activity was visually-evaluated.

## Evaluation of antimicrobial activity

The propylene glycol was stored at -20°C until the day of the test. Before testing, the aerobic isolates were scraped from frozen vials on Trypticase Soy agar and incubated overnight at 35°C. The anaerobic isolates were scraped from frozen vials on enriched Brucella Agar (containing haemin 5 $\mu$g/ml, vitamin K 1 $\mu$g/ml and 5% lysed sheep blood) and incubated at 35°C under anaerobic conditions for 48 hours. The yeast isolates were scraped from frozen vials on Sabouraud dextrose agar and incubated overnight at 35°C. The fungal isolates were grown on potato dextrose agar and incubated for 7-10 days at 35°C. The colonies were sampled from the respective medium, re-suspended in the appropriate culture medium, splitted into aliquots and stored at -80°C.

For tests on aerobes, the Mueller Hinton II medium (MHBII; BD 212322, Batch 0257287; Becton, Dickinson and Co., Sparks, MD) was used. For tests on anaerobes, the Brucella medium (SBB, BD 211088, Batch 0320091; Becton, Dickinson and Co., Sparks, MD) enriched with haemin 5 $\mu$g/ml (H9039, batch 099K1183; Sigma-Aldrich, St. Louis, MO), vitamin K 1 $\mu$g/ml (V3501; Batch 106K1523; Sigma-Aldrich, St. Louis, MO) and 5% horse blood (Batch 117342-2; Cleveland Scientific) was used. The isolates of both fungi and yeasts were tested in RPMI medium (SH30011.04; Batch AWA92121B; HyClone Labs, Logan, UT) buffered with MOPS (475898; Batch D00093780; CalBiochem, La Jolla, CA) 0.165 M. II pH of the RPMI medium was adjusted to 7.0 with NaOH 1 N. the medium was subjected to sterile filtration using a 2 $\mu$m PES filter and stored at 4°C until the time of use.

The test was performed in accordance with the methods described by the Clinical and Laboratory Standards Institute (CLSI). The initial concentration of propylene glycol was 40%. Serial dilutions of the solvent were performed manually on 96-well plates. The wells located at columns 2-12 were filled up with 460 $\mu$l of sterile water; then, 460 $\mu$l of the solvent were added to the well located at column 2 of the "mother" plate and mixed. The solvent was then serially diluted and each one of properly diluted aliquot was added to the relevant plate up to column 11. Column 12 contained sterile water, as a control of the growth of the microorganism. The "daughter" plates were 96-well standard microdilution plates (Costar 3795) and were filled up with 50 μl of the medium suitable for each micro-organism. 40 μl solvent and ef the samples obtained from serial dilutions prepared in the "mother" plate were added to the "daughter" plates.

For each micro-organism, a standard inoculum was prepared according to the CSLI methods. For the fungi and bacteria, the colonies were sampled from the agar plates and suspended in MHBII (aerobes), SBB (anaerobes) or RPMI (yeasts) at a McFarland standard of 0.5. For *Aspergillus niger* 624, 0.5 ml of 0.85% saline solution was put on the agar, which had been inoculated 7-10 days before, and a suspension of the fungus on the surface of the agar was prepared. The resultant mixture was transferred into a sterile test tube and allowed to rest for 5 minutes to deposit the heaviest particles. The supernatant was removed, placed in another test tube and spun for 15 seconds. The density was adjusted to McFarland turbidity standard 0.5. For each microorganism, dilutions were carried out in the appropriate medium to achieve the cell concentration described in the CLSI methodology. 10 μl of suspension containing each microorganism was then inoculated into each well. At the end of the procedure, the wells of the "daughter" plates contained 50 μl of medium, 40 μl of propylene glycol and 10 μl of inoculum. The plates were then covered with a lid. The ones containing the aerobes, fungi and yeasts were placed in plastic bags and incubated at 35°C for approximately 24-48 hours before reading. The ones containing the anaerobes were placed into BD GasPak chambers and incubated at 35°C for 46-48 hours under anaerobic conditions. The plates were then visualised from below using a plate visualiser. The growth inhibition was read and recorded as the lowest concentration of solvent which inhibits the visible growth of the organism.

Statistical analysis

The values of cell proliferation, collagen production, and production of inflammatory cytokines obtained from experiments were subjected to statistical analysis with the OpenStat program version 26.03.2012 (www.statprograms4u.com). In particular, comparison between mean values was carried out using Student's two-tailed *t*-test. Values of $P \leq 0.05$ were considered statistically significant.

## RESULTS OF THE BIOASSAYS AND ANTI-RADICAL ACTIVITY

The results of the test to aimed at evaluating the effect of the formulations of the invention on mitochondrial dehydrogenase activity are shown in the plots reported below, in which the mitochondrial dehydrogenase activity expressed as the percentage of activity is defined as:

$$\text{Mitochondrial dehydrogenase activity\%} = ((\text{absorbance of the treated cells} - \text{absorbance of the blank}) / (\text{absorbance of the control} - \text{absorbance of the blank})) \times 100$$

In the graphs Illustrated in Figs. 1-4, the mitochondrial dehydrogenase activity % (y-axis) is reported versus concentrations of the substances (x-axis). The results of the test aimed at evaluating the effect of the formulations of the invention on cell migration are given in Table 3.

**Table 3.** Effect of four formulations of the invention on the migration of HDF cells at a concentration of 0.01% w/v

| ID Example | Percentage area of HDF cells ± SD at 24 h | Percentage area of HDF cells ± SD at 48 h |
|---|---|---|
| Control | $32 \pm 1$ | $48 \pm 4$ |
| Formulation 1 | $45 \pm 9$ | $71 \pm 1$ |
| Formulation 2 | $41 \pm 5$ | $71 \pm 3$ |
| Formulation 3 | $33 \pm 1$ | $67 \pm 7$ |
| Formulation 4 | $36 \pm 14$ | $76 \pm 11$ |

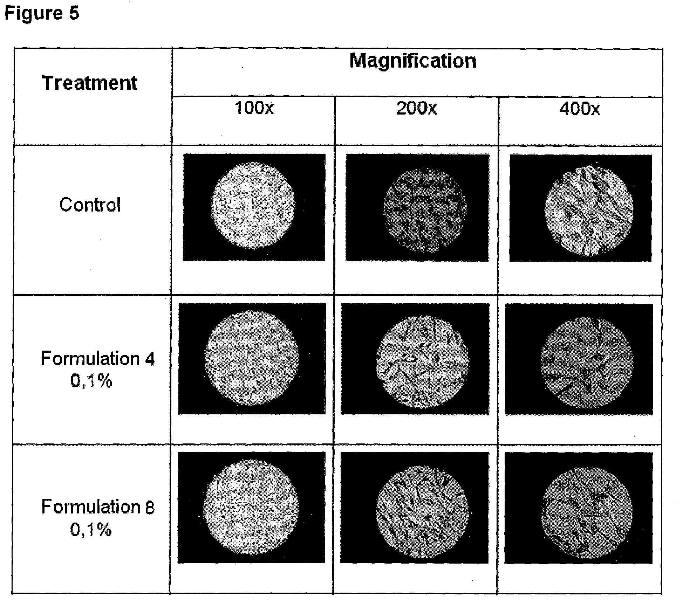

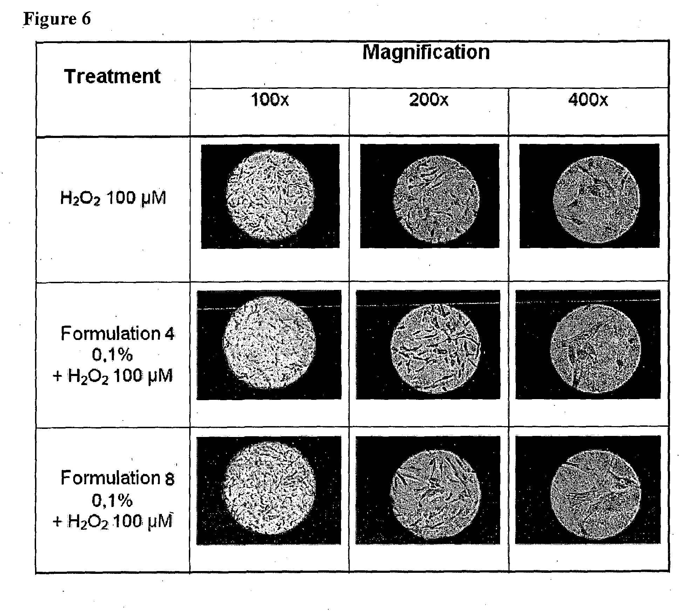

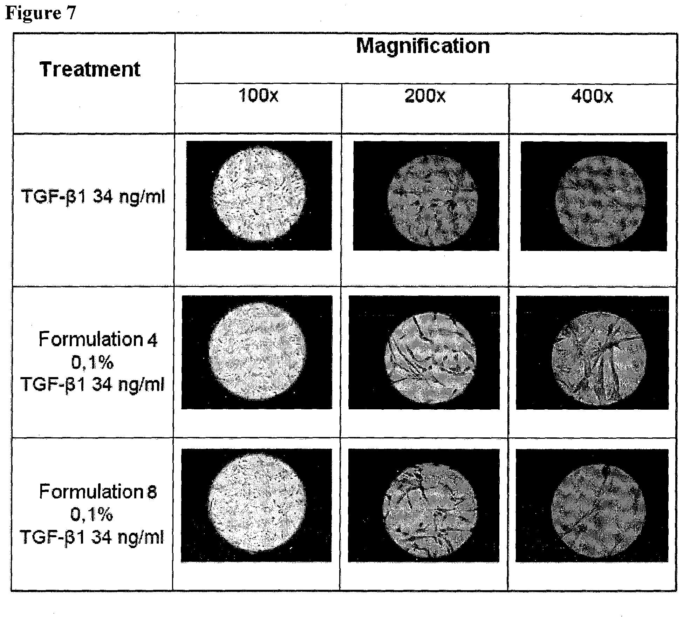

The effect of the treatment of the formulations 4 and 8, for 24 hours at the specified concentrations, on the morphology of HDF cells stained for the collagen with PSR are given in Figs. 5-7. These figures show the data obtained from HDF grown under standard growth conditions, in the presence of 100 μM $H_2O_2$ and in the presence of 34 ng/ml TGF-β1, respectively. The cell morphology was evaluated using an optical microscope at 100x, 200x e 400x magnifications.

For what concerns cell proliferation data and the data, normalised for the number of cells, which refer to collagen, IL-1β, and IL-6 production, the results are reported in Tables 4 and 5, respectively.

**Table 4.** Data concerning cell proliferation, collagen production and the release of inflammatory cytokines obtained from HDF collagen-stained with Sirius Red and subjected to various treatments with the formulations 4 and 8 at the specified concentrations, for a period of 24 hours, under standard growth conditions, in the presence of 100 $\mu$M $H_2O_2$ and in the presence of 34 ng/ml TGF-$\beta$1. The cell proliferation and the production of collagen are expressed as the mean value of three experiments $\pm$ SD, while the production of IL-1$\beta$ and IL-6 inflammatory cytokines is expressed as the mean value of two experiments $\pm$ SD.

| Treatment | Standard conditions | | | | 100 $\mu$M $H_2O_2$ | | | | 34 ng/ml TGF-$\beta$1 | | | |
|---|---|---|---|---|---|---|---|---|---|---|---|---|
| | No. of photo cells[a] $\pm$ SD | Abs*10[6]/No. of photo cells[b] $\pm$ SD | IL-1$\beta$ ng/(ml* No. of photo cells)[c] $\pm$SD | IL-6 ng/(ml* No. of photo cells)[d] $\pm$ SD | No. of photo cells[a] $\pm$ SD | Abs*10[-6]/No. of photo cells[b] $\pm$SD | IL-1$\beta$ ng/ (ml* No. of photo cells)[c] $\pm$SD | IL-6 ng/(ml* No. of photo cells)[d] $\pm$ SD | No. of photo cells[a] $\pm$ SD | Abs*10[-6]/No. of photo cells[b] $\pm$SD | IL-1$\beta$ ng/ (ml* No. of photo cells)[c] $\pm$SD | IL-6 ngl(ml* No. of photo cells)[d] $\pm$ SD |
| Control (C) | 186$\pm$28 | 1720$\pm$102 | 112$\pm$1 | 715$\pm$32 | 183$\pm$17 | 1475$\pm$16 | 142$\pm$2 | 1295$\pm$109 | 163$\pm$4 | 2576$\pm$122 | 159$\pm$6 | 969$\pm$36 |
| Formulation 40.1% | 210$\pm$38 | 1142$\pm$47 | 102$\pm$10 | 785$\pm$4 | 155$\pm$36 | 2174$\pm$58 | 107$\pm$7 | 1265$\pm$40 | 176$\pm$16 | 2284$\pm$272 | 113$\pm$10 | 676$\pm$18 |
| Formulation 80.1% | 199$\pm$22 | 1206$\pm$90 | 111$\pm$1 | 803$\pm$8 | 142$\pm$5 | 2711$\pm$359 | 120$\pm$8 | 1219$\pm$98 | 171$\pm$23 | 2345$\pm$23 | 115$\pm$5 | 660$\pm$15 |

[a] No. of photo cells = number of cells read by microphotography, a parameter indicating cell proliferation
[b] Abs*10[-6]/No. of photo cells = absorbance scaled for a factor of 10[-6] as a ratio of the number of cells read by microphotography, a parameter indicating the production of collagen per cell
[c] IL-1$\beta$ ng/(ml*No. of photo cells) = concentration of IL-1$\beta$ expressed in ng/ml as a ratio of the number of cells read by microphotography, a parameter indicating the production of IL-1$\beta$ per cell
[d] IL-6 ng/(ml*No. of photo cells) = concentration of IL-6 expressed in ng/ml as a ratio of the number of cells read by microphotography, a parameter indicating the production of IL-6 per cell
SD = standard deviation

**Table 5.** Comparison between the mean values for cell proliferation, collagen production and release of inflammatory cytokines shown in Table 4, expressed as a probability (P) obtained using Student's t-test. The probability values $\leq 0.05$ are shown in **bold typeface.**

| Treatment | Standard conditions-value of P vs. control | | | | 100 $\mu$M $H_2O_2$ - value of P vs. control + $H_2O_2$ | | | | 34 ng/ml TGF-$\beta$1 - value of P vs. control + TGF-$\beta$1 | | | |
|---|---|---|---|---|---|---|---|---|---|---|---|---|
| | No. of photo cells[a] | Abs*10-6/No. of photo cells[b] | IL-1$\beta$ ng/ (ml* No. of photo cells)[c] | IL-6 ng/ (ml* No. of photo cells)[d] | No. of photo cells[a] | Abs*10-6/No. of photo cells[b] | IL-1$\beta$ ng/ (ml* No. of photo cells)[c] | IL-6 ng/ (ml* No. of photo cells)[d] | No. of photo cells[a] | Abs*10-6/No. of photo cells[b] | IL-1$\beta$ ng/ (ml* No. of photo cells)[c] | IL-6 ng/ (ml* No. of photo cells)[d] |
| Control (C) | - | - | - | - | - | - | - | - | - | - | - | - |
| Control + $H_2O_2$ | 0.8816 | 0.0147 | 0.0028 | 0.0186 | - | - | - | - | - | - | - | - |
| Control + TGF-$\beta$1 | 0.2318 | 0.0007 | 0.0083 | 0.0175 | - | - | - | - | - | - | - | - |
| Formulation 40.1% | 0.4282 | 0.0009 | 0.0032 | 0.0948 | 0.2901 | P<0.0001 | 0.021 | 0.7498 | 0.2439 | 0.165 | 0.0307 | 0.0093 |
| Formulation 80.1% | 0.5615 | 0.0028 | 0.0306 | 0.3597 | 0.016 | 0.004 | 0.0636 | 0.5397 | 0.5848 | 0.0322 | 0.0154 | 0.0079 |

[a] No. of photo cells = number of cells read by microphotography, a parameter indicating cell proliferation

[b] Abs*10-6/No. of photo cells = absorbance scaled for a factor of 10-6 as a ratio of the number of cells read by microphotography, a parameter indicating the production of collagen per cell

[c] IL-1$\beta$ ng/(ml*No. of photo cells) = concentration of IL-1$\beta$ expressed in ng/ml as a ratio of the number of cells read by microphotography, a parameter indicating the production of IL-1$\beta$ per cell

[d] IL-6 ng/(ml*No. of photo cells) = concentration of IL-6 expressed in ng/ml as a ratio of the number of cells read by microphotography, a parameter indicating the production of IL-6 per cell SD = standard deviation

The results of assays aimed at estimating potential anti-radical activity of the formulations of the invention are shown in Table 6.

**Table 6.** Anti-radical activity.

| Substance | Anti-radical activity |
|---|---|
| **Controls** | |
| Negative control with $H_2O_2$ | Inactive |
| Positive control with vitamin C | Active |
| **Formulations containing *C. majus* extracts** | |
| Formulation 4 | Active |
| Formulation 8 | Active |

The results of assays aimed at estimating potential antimicrobial activity of the propylene glycol are given in Table 7. This table shows the concentrations of propylene glycol which inhibit the growth of each of the microorganisms tested expressed as percentage.

**Table 7.** Antimicrobial activity of the propylene glycol

| Microorganism | Percentage of propylene glycol which inhibits microorganism growth |
|---|---|
| *Staphylococcus aureus* 100 (ATCC 29213)[1] | 20 |
| *Staphylococcus aureus* 2119 (USA 100 MRSA)[1] | 20 |
| *Escherichia coli* 102 (ATCC 25922)[1] | 10 |
| *Pseudomonas aeruginosa* 103 (ATCC 27853) 1 | 10 |
| *Corynebacterium striatum* 5975 (ATCC 6940)[1] | 20 |
| *Bacteroides fragilis* 123 (ATCC 25285)[2] | 10 |
| *Finegoldia magna* 5976 (ATCC 29328) | >40 |
| *Propionibacterium acnes* 1286 (ATCC 11829)[2] | 5 |
| *Candida albicans* 104 (ATCC 90028)[3] | 10/10 |
| *Candida parapsilosis* 2323 (ATCC 22019)[3] | 10/10 |
| *Aspergillus niger* 624 (ATCC 16888 )[2] | 10 |
| Plates read after 19 hours incubation<br>[2] Plates read after 46-48 hours incubation<br>[3] Plates read after 24 and 48 hours incubation | |

From the detailed description and from the examples mentioned above, the advantages arising from the compositions of the present invention are obvious. In particular, such compositions have shown themselves to be surprisingly and advantageously well suited for the use in the cosmetic and in the pharmaceutical fields, and in particular for use in the treatment of skin lesions characterised by epidermal hyperproliferation and dermal damage, possibly associated with a microbial infection.

**Claims**

1. Composition comprising a *Chelidonium majus* extract and *Copaiba.*

2. Composition according to claim 1, wherein said composition comprises berberine and beta-caryophyllene, wherein the ratio between the amount in weight of beta-caryophyllene and the amount in weight of berberine is within the range from 7,000 to 60,000,000.

3. Composition according to any one of claims 1 or 2, wherein said *Chelidonium majus* extract comprises protopine, stylopine, chelidonine, sanguinarine, berberine, chelerythrine and coptisine and wherein:

   - the ratio between the amount in weight of stylopine and the total amount in weight of sanguinarine, chelerythrine, chelidonine and protopine is greater than or equal to 5;
   - the ratio between the amount in weight of berberine and the total amount in weight of sanguinarine, chelerythrine, chelidonine, protopine is greater than or equal to 1; and
   - the ratio between the amount in weight of berberine and the amount in weight of coptisine in the *Chelidonium majus* extract is greater than or equal to 0.05.

4. Composition according to any one of claims 1 to 3, further comprising *Rosa mosqueta*

5. Composition according to any one of claims 1 to 4, further comprising a silicone.

6. Composition according to claim 5, wherein in said composition the ratio between the amount in weight of beta-caryophyllene and the amount in weight of silicone compound is within the range from 10 to 40.

7. Composition according to any one of claims 1 to 6, comprising propylene glycol.

8. Composition according to any one of claims 5 or 6, wherein said silicone compound is dimethicone.

9. Composition according to any one of claims 1 to 7, for use in the treatment of skin lesions **characterised by** epidermal hyperproliferation and dermal damage.

10. Composition for use according to claim 9 wherein said skin lesions **characterised by** epidermal hyperproliferation and dermal damage are selected from the group consisting of acne, dermatitis, psoriasis, skin dyschromia and keratosis.

11. Composition for use according to claim 10, wherein said skin lesions **characterised by** epidermal hyperproliferation and dermal damage are selected from the group consisting of acne and keratosis.

**Patentansprüche**

1. Zusammensetzung mit einem Chelidonium-majus-Exrakt und Copaiba.

2. Zusammensetzung gemäß Anspruch 1, wobei die Zusammensetzung Berberin und Beta-Caryophyllen aufweist, wobei das Gewichtsverhältnis von Beta-Caryophyllen zu Berberin im Bereich von 7.000 bis 60.000.000 liegt.

3. Zusammensetzung gemäß einem der Ansprüche 1 oder 2, wobei der Chelidonium-majus-Exrakt Protopin, Stylopin, Chelidonin, Sanguinarin, Berberin, Chelerythrin und Coptisin aufweist und wobei:

   - das Verhältnis der Gewichtsmenge von Stylopin zur Gesamtgewichtsmenge von Sanguinarin, Chelerythrin, Chelidonin und Protopin größer oder gleich 5 ist;
   - das Verhältnis der Gewichtsmenge von Berberin zur Gesamtgewichtsmenge von Sanguinarin, Chelerythrin, Chelidonin und Protopin größer oder gleich 1 ist und
   - das Verhältnis der Gewichtsmenge von Berberin zur Gewichtsmenge von Coptisin in dem Chelidonium-majus-Exrakt größer oder gleich 0,05 ist.

4. Zusammensetzung gemäß einem der Ansprüche 1 bis 3, weiter aufweisend Rosa mosqueta.

**5.** Zusammensetzung gemäß einem der Ansprüche 1 bis 4, weiter aufweisend ein Silikon.

**6.** Zusammensetzung gemäß Anspruch 5, wobei in der Zusammensetzung das Gewichtsverhältnis von Beta-Caryophyllen zur Silikonverbindung im Bereich von 10 bis 40 liegt.

**7.** Zusammensetzung gemäß einem der Ansprüche 1 bis 6, aufweisend Propylenglycol.

**8.** Zusammensetzung gemäß einem der Ansprüche 5 oder 6, wobei die Silikonverbindung Dimethicon ist.

**9.** Zusammensetzung gemäß einem der Ansprüche 1 bis 7 zur Verwendung bei der Behandlung von Hautläsionen charakterisiert durch epidermale Hyperproliferation und Hautschäden.

**10.** Zusammensetzung zur Verwendung gemäß Anspruch 9, wobei die durch epidermale Hyperproliferation und Hautschäden charakterisierten Hautläsionen ausgewählt werden aus der Gruppe bestehend aus Akne, Dermatitis, Psoriasis, Haut-Dyschromie und Keratose.

**11.** Zusammensetzung zur Verwendung gemäß Anspruch 10, wobei die durch epidermale Hyperproliferation und Hautschäden charakterisierten Hautläsionen ausgewählt werden aus der Gruppe bestehend aus Akne und Keratose.

**Revendications**

**1.** Composition comprenant un extrait de *Chelidonium majus* et du copahu.

**2.** Composition selon la revendication 1, dans laquelle ladite composition comprend de la berbérine et du bêta-caryophyllène, dans laquelle le rapport entre la quantité en poids de bêta-caryophyllène et la quantité en poids de berbérine se situe dans la plage de 7 000 à 60 000 000.

**3.** Composition selon l'une quelconque des revendications 1 ou 2, dans laquelle ledit extrait de *Chelidonium majus* comprend de la protopine, de la stylopine, de la chélidonine, de la sanguinarine, de la berbérine, de la chélérythrine et de la coptisine et dans laquelle :

- le rapport entre la quantité en poids de stylopine et la quantité totale en poids de sanguinarine, chélérythrine, chélidonine et protopine est supérieur ou égal à 5 ;
- le rapport entre la quantité en poids de berbérine et la quantité totale en poids de sanguinarine, chélérythrine, chélidonine, protopine est supérieur ou égal à 1 ; et
- le rapport entre la quantité en poids de berbérine et la quantité en poids de coptisine dans l'extrait de *Chelidonium majus* est supérieur ou égal à 0,05.

**4.** Composition selon l'une quelconque des revendications 1 à 3, comprenant en outre *Rosa mosqueta.*

**5.** Composition selon l'une quelconque des revendications 1 à 4, comprenant en outre un silicone.

**6.** Composition selon la revendication 5, dans laquelle dans ladite composition, le rapport entre la quantité en poids de bêta-caryophyllène et la quantité en poids de composé de silicone se situe dans la plage de 10 à 40.

**7.** Composition selon l'une quelconque des revendications 1 à 6, comprenant du propylène glycol.

**8.** Composition selon l'une quelconque des revendications 5 ou 6, dans laquelle ledit composé de silicone est le diméthicone.

**9.** Composition selon l'une quelconque des revendications 1 à 7, pour une utilisation dans le traitement de lésions cutanées **caractérisées par** une hyperprolifération épidermique et des lésions dermiques.

**10.** Composition pour une utilisation selon la revendication 9, dans laquelle lesdites lésions cutanées **caractérisées par** une hyperprolifération épidermique et des lésions dermiques sont choisies dans le groupe constitué de l'acné, la dermatite, le psoriasis, la dyschromie cutanée et la kératose.

11. Composition pour une utilisation selon la revendication 10, dans laquelle lesdites lésions cutanées **caractérisées par** une hyperprolifération épidermique et des lésions dermiques sont choisies dans le groupe constitué de l'acné et la kératose.

**Figure 1a**

**Figure 1b**

**HDF_42h**

◆ EG 42h
□ Rose_oil 42h

△ Copaiba 42h
▽ TM 42h

**Figure 2**

HDF

Formulation 1
Formulation 2
Formulation 3
Formulation 4
Formulation 5
Formulation 6
Formulation 7
Formulation 8

**Figure 3**

HEK

**Figure 4**

**HUVEC**

Legend:
- ■ Formulation 1
- ▲ Formulation 2
- ▼ Formulation 3
- ◆ Formulation 4
- ● Formulation 5
- □ Formulation 6
- △ Formulation 7
- ▽ Formulation 8

X-axis: Concentration (log % w/v)
Y-axis: Mitochondrial dehydrogenase activity %

## Figure 5

| Treatment | Magnification | | |
|---|---|---|---|
| | 100x | 200x | 400x |
| Control | | | |
| Formulation 4 0,1% | | | |
| Formulation 8 0,1% | | | |

Figure 6

| Treatment | Magnification | | |
|---|---|---|---|
| | 100x | 200x | 400x |
| H₂O₂ 100 µM | | | |
| Formulation 4 0,1% + H₂O₂ 100 µM | | | |
| Formulation 8 0,1% + H₂O₂ 100 µM | | | |

Figure 7

| Treatment | Magnification | | |
|---|---|---|---|
| | 100x | 200x | 400x |
| TGF-β1 34 ng/ml | | | |
| Formulation 4 0,1% TGF-β1 34 ng/ml | | | |
| Formulation 8 0,1% TGF-β1 34 ng/ml | | | |

## REFERENCES CITED IN THE DESCRIPTION

### Patent documents cited in the description

- WO 09112226 A
- US 20060045930 A
- WO 2009112226 A
- DE 4031960
- FR 2661330

### Non-patent literature cited in the description

- **HERNANDEZ - MARTINEZ.** Repair, regeneration and fibrosis. Lippincott, 1999
- **SATISH L ; KATHJU S.** Dermatology Research and Practice. 2010, 11
- **BRUNI A.** Farmacognosia generale e applicata. *General and applied pharmacognosy,* 1999, 300-301
- **KIM HK et al.** *Journal of Pharmaceutical Sciences,* 1969, vol. 58, 372-374
- **SLAVIK J ; SLAVIKOVA L.** *Collection of Czechoslovak Chemical Communications,* 1977, vol. 42, 2686-2693
- **SLAVIK J et al.** *Collection of Czechoslovak Chemical Communications,* 1965, vol. 30, 3697-3704
- **TIN-WA M et al.** *Journal of Natural Products,* 1972, vol. 35, 87-89
- **GU Y et al.** *Journal of Separation Science,* 2010, vol. 33, 1004-1009
- **AMENTA R et al.** *Fitoterapia [Phytotherapy,* 2000, vol. 71 (1), S13-S20
- *CHEMICAL ABSTRACTS,* 9000-12-8 / 8001-61-4
- **VEIGA JUNIOR VF et al.** *Journal of Ethnopharmacology,* 2007, vol. 112, 248-254
- **OLIVEIRA DOS SANTOS A et al.** *Mem Inst Oswaldo Cruz,* 2008, vol. 103, 277-281
- **SARKOZI A et al.** *Chromatographia,* 2006, vol. 63, 81-86
- *Am. J. Physiol. Renal. Physiol.,* 2007
- **CARPENTER AE et al.** *Genome Biology,* 2006, vol. 7, R100